# EUROPEAN PATENT APPLICATION

(11) **EP 3 822 633 A1**
(43) Date of publication of application: **19.05.2021**
(21) Application number: 19209127.0
(22) Date of filing: 14.11.2019
(51) Int. Cl.: G01N 33/24, G02B 27/01, G06F 3/01

(54) **COMPUTER ENHANCED SOIL SAMPLE TESTING METHOD AND APPARATUS**

(71) Applicant: University College Dublin, Dublin 4 (IE)
(72) Inventor: Zheng, Mengya, Co. Dublin, (IE); Campbell, Abraham, Dublin 4 (IE)
(74) Representative: Purdylucey Intellectual Property

(57) **Abstract**

A method for determining physical attributes of a geographical area of interest by determining the physical location of the area of interest, creating a representation of the area of interest on a graphical user interface of a portable computing device, mapping a graphical object onto a location in the representation of the area of interest, the graphical object representing a location in the area of interest where a task is to be undertaken and recording the performance of the task. The method uses a location detector such as a smart phone to provide data to a VR headset which can combine the representation of the location with the graphical object.

## Description

### Field of the Invention

The invention relates to a computer enhanced system and method for determining physical attributes of a geographical area of interest and in particular for collecting soil samples for testing

### Background to the Invention

Basic plant nutrition requires the presence of nutrients such as nitrogen, phosphorus and potassium in soil.

Soil sampling is the process of taking a small sample of soil, which is then sent to a lab to determine the nutrient content, which is one a measure of soil quality Soil sampling can also determine the pH levels of the soil, available lime, complete sulphur content and total CaCO₃.

Soil analysis may test the chemical, physical and biological properties, which are critical to plant nutrition. The principle of soil analysis is to determine the average nutrient status of an area and to give a measure of the available nutrients in the soil. The analysis is based upon a number of samples which are taken at different locations each of which comprise 0.25 - 0.5 kg of soil and taken to represent the entire sampling area.

The determination of the levels of available nutrients present in the soil allows a farmer to manage the fertility of the soil by, for example, adding more nutrients to the soil as well as more accurately determining which crops would yield the best growth in that particular soil.

The results of a soil analysis are only as good as the sample on which it is based. A soil sample which is to be analysed must be representative of the area sampled and be taken to a uniform depth. Sampling requires the use of a suitable soil corer and the taking of a sample to the correct sampling depth which is typically 10 cm.

Separate samples must be taken from areas that are different in soil type, with different cropping histories, slopes, drainage or persistent poor yields.

A common approach to ensuring that the sample is representative, is to sample the soil in positions along a predetermined surface pattern in the sample area, for example, a W-shaped pattern is commonly used for this purpose.

However, it is notoriously difficult to accurately follow a surface pattern across a field and the sampler may often veer away from the correct sample position on the sample pattern. This leads to samples being taken which do not match the locations where previous samples had been taken. In addition, the shape and location of the pattern may be different year on year due to human error.

Therefore, sampling is an inaccurate manual process which is also time consuming. This is particularly the case for large farms where the process also suffers from a shortage of staff.

Some current systems allow the use of smartphone- based apps to assist zone navigation. However, there are multiple limitations associated with these processes. They are labour intensive in terms of planning and in terms of carrying out sampling and the use of the smartphone in hand along with the carrying of sampling equipment is burdensome.

### Summary

It is an object of the present invention to provide an accurate and reliable method and apparatus for ensuring that samples are taken in a reliable and repeatable way to ensure the accuracy and reliability of the sample test results.

In accordance with one aspect of the invention there is provided, as set out in the appended claims, a method for determining physical attributes of a geographical area of interest, the method comprising the steps of:
determining the physical location of the area of interest;
creating a representation of the area of interest on a graphical user interface of a portable computing device;
mapping a graphical object onto a location in the representation of the area of interest, the graphical object representing a location in the area of interest where a task is to be undertaken; and
recording the performance of the task.

In one embodiment, the geographical area is a field.

In this example of the present invention, the location of the field is determined using Global Positioning System (GPS) location data which is obtained by the smartphone. The GPS data is made available to the headset via a wireless communication system such as Bluetooth®.

Alternatively, after an initial GPS "registration", location of the user is tracked using the vision tracking capabilities of the Vive Focus or similar headsets.
In one embodiment, the step of measuring the physical attributes comprises taking a soil sample.

In one embodiment, the step of creating a representation of the area of interest on a graphical user interface of a portable computing device comprises acquiring the GPS position of the area of interest.

In one embodiment of the invention, the step of creating a representation of the area of interest on a graphical user interface comprises the creation of points of interest within the area of interest.

In one embodiment, the location of the points of interest is calculated to define optimum locations in the geographical area at which the task is undertaken.

In one embodiment of the invention, the points of interest are sampling points where a soil sample is to be taken.

In one embodiment of the invention, the GPS position is acquired using a smartphone.

In one embodiment, the computing device is an Augmented Reality (AR) headset.

In one embodiment, the computing device is a Virtual Reality (VR) headset.

In one embodiment, the computing device is a Mixed Reality (MR) headset.

In one embodiment, the computing device is a portable computing device such as a tablet or a hand-held computing device such as a smartphone.

In one embodiment of the invention, the computing device comprises a combination of an augmented reality device and a smartphone, wherein the smartphone provides location information to the augmented reality headset.

In one embodiment of the invention, the computing device comprises a combination of a virtual reality device and a smartphone, wherein the smartphone provides location information to the virtual reality headset.

In one embodiment of the present invention, the graphical object is mapped onto the points of interest.

In one embodiment, the graphical object is mapped onto the points of interest to create a graphical representation of the points of interest which overlays the representation of the area of interest.

In one embodiment, the graphical object represents is a continuous path.

In one embodiment, the graphical object represents one or more discrete location.

In one embodiment, the graphical object represents the position on the ground to which the device must be moved and from where the task must be performed.

In one embodiment, the step of recording the performance of the task comprises recording on the computing device that the task has been performed.

In one embodiment, the step of recording the performance of the task comprises using voice recognition to record the response of a user, said response indicating that the task has been performed.

In one embodiment, performance of the task is logged then the method defines a location in the area of interest where a subsequent task is to be undertaken.

In one embodiment, the performance of the task is recorded using a mobile tag which links the performed task with the location at which the task was performed.

In one embodiment, the tag is a barcode.

In one embodiment the tag is a matrix barcode, for example a QR Code®.

In one embodiment, the task comprises sampling soil.

In one embodiment, the sample is contained in a bar-coded bag which is scanned to link the bag and the acquired sample to the location.

In accordance with a second aspect of the invention there is provided a device for determining physical attributes of a geographical area of interest as defined in the method of the first aspect, the device comprising:
a location detector for determining the physical location of the area of interest;
a portable computing device which
creates a representation of the area of interest on a graphical user interface of a portable computing device based on the physical location determined by the location detector;
creates a mapping of a graphical object onto a location in the representation of the area of interest, the graphical object representing a location in the area of interest where a task is to be undertaken; and
a data recorder for recording the performance of the task.

In one embodiment, the computing device is an Augmented Reality (AR) headset.

In one embodiment, the computing device is a Virtual Reality (VR) headset.

In one embodiment, the computing device is a Mixed Reality (MR) headset.

In one embodiment, the computing device is a portable computing device such as a tablet or a hand-held computing device such as a smartphone.

In one embodiment of the invention, the computing device comprises a combination of an augmented reality device and the location detector is a smartphone, wherein the smartphone provides location information to the augmented reality headset.

In one embodiment of the invention, the computing device comprises a combination of an virtual reality device and a smartphone, wherein the smartphone provides location information to the virtual reality headset.

In one embodiment of the present invention, the graphical object is mapped onto the points of interest.

In one embodiment, the data recorder records the performance of the task on the computing device.

In one embodiment, the data recorder uses voice recognition to record the response of a user, said response indicating that the task has been performed.

In one embodiment, performance of the task is logged then the method defines a location in the area of interest where a subsequent task is to be undertaken.

In one embodiment, the data recorder further comprises a mobile tag which links the performed task with the location at which the task was performed.

In one embodiment, the tag is a barcode.

In one embodiment the tag is a matrix barcode, for example a QR Code®.

In one embodiment, the task comprises sampling soil.

In one embodiment, the sample is contained in a bar-coded bag which is scanned to link the bag and the acquired sample to the location.

In accordance with a third aspect of the invention there is provided a computer system comprising program instructions for the operation of the method in accordance with the first aspect of the invention.

### Brief Description of the Drawings

The invention will be more clearly understood from the following description of an embodiment thereof, given by way of example only, with reference to the accompanying drawings, in which:-
Figure 1 is a schematic diagram of a first example of a system in accordance with the present invention;
Figure 2 is a schematic diagram of a second example of a system in accordance with the present invention;
Figure 3 is a flow diagram which shows an example of the method of the present invention;
Figure 4 is a flow diagram which shows a second example of the method of the present invention;
Figure 5 is an example of a graphical user interface in accordance with the present invention.

### Detailed Description of the Drawings

The invention provides a novel way to sample soil by defining locations or points of interest and creating an optimum walking route between them.

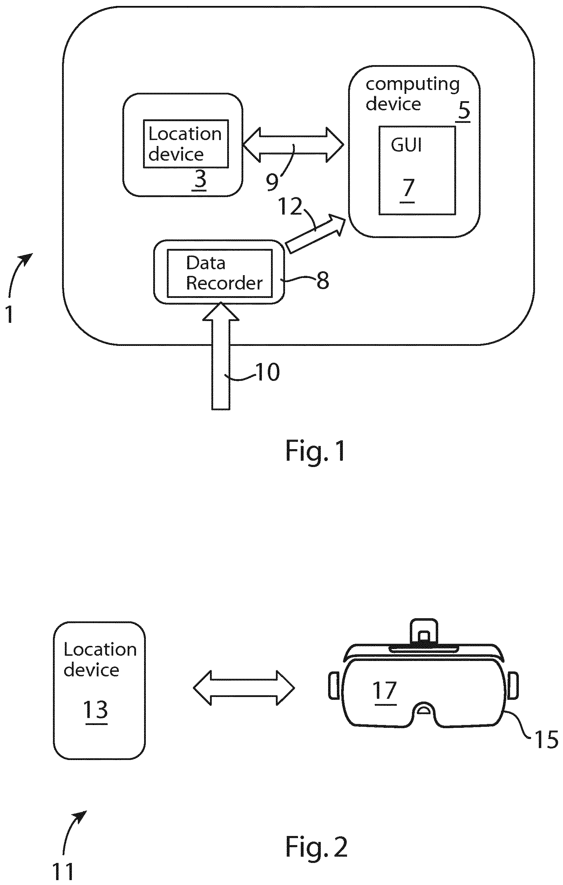

Figure 1 illustrates an example of a system 1 for implementing the method in accordance with the present invention. In this example, the system is an integrated device which contains a location device 3 which is capable of receiving and interpreting location information so as to accurately determine the location of the device. A computing device 5 is provided with a graphical user interface 7 which displays a combination of location information and a graphical object which shows a path to the location at which a sample is to be taken. A voice recorder is provided in the location device.

Figure 2 illustrates a system 11 which comprises a smartphone 13 and a computing device 15 which has a graphical user interface 17. The computing device may be an AR/VR device or another device which can map graphical information onto a location.

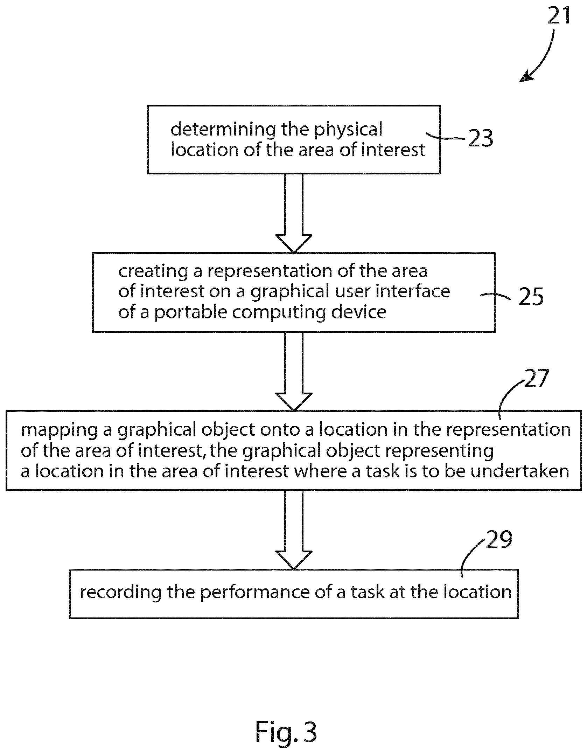

Figure 3 shows an example of a method 21 in accordance with the present invention. The method firstly determines the physical location of the area of interest 23, then creates a representation of the area of interest on a graphical user interface of a portable computing device 25. Thereafter, a graphical object is mapped 27 onto a location in the representation of the area of interest, the graphical object representing a location in the area of interest where a task is to be undertaken, once the task has been performed, the method of the present invention records the performance of the task 29.

In the case of the specific application of the invention to soil sampling, this combination of steps allows the user to identify soil parameters by reference to a sampling point using location information from a smartphone and then to present the sampling point in visual heads-up form on an AR/VR device. The use of heads-up technology allows the user to have free hands. Such soil sampling is required in precision agriculture in order to establish field management zones for variable rate applications. Advantageously the present invention cuts the time involved in planning and executing sampling.

The following examples describe an application of the invention in which the graphical object comprises a sampling pattern which is mapped on to a representation of a field zone which has been created using GPS and satellite data. The representation of the field is created by acquiring the field location through an application programming interface (API) to connect to a software application which provides location and aerial information such as satellite images.

The method also acquires a set of areas or points of interest which are the projected sampling points. The system then calculates the optimum route for sampling and displays this visually as a graphical object to the user in an AR or VR headset.

In one example, the graphical object is a pattern which places the letter "W" on top of the representation of a field or similar area of land. In use, a user follows the pattern using a VR or AR headset which displays the optimum route through the zone by staying on the path defined by the letter "W" and acquiring samples at specific locations as instructed by the device.

In this example of the present invention, the location of the field is determined using Global Positioning System (GPS) location data which is obtained by the smartphone. The GPS data is made available to the headset via a wireless communication system such as Bluetooth®.

Alternatively, after an initial GPS "registration", the location of the user is tracked using the vision tracking capabilities of a VR headset, for example, the Vive Focus VR headset. The user is presented in their visual field with an object which in one embodiment is a red cube on the ground representing the position in which they need to move to in order to commence sampling. Once they reach that spot, the system tells the user they are at the correct spot. The user takes out a sample bag with a QR code on it, acquires the soil sample and then scans the QR code on the bag thus linking the acquired sample to that location. A data recorder is used to record completion of the task. In this example, the data recorder is a voice recorder such that the user communicates by voice command with the system by saying " Got my sample". That terminates the first (or subsequent) sample operation(s) and then the display shows the user the next red cube where the next sample is to be taken.

In at least one embodiment, the system employs conventional QR technology and allows a sample to be tracked off-line and used in any order thus improving speed of operation.

In addition, it allows the time stamp to be captured. Recording an image of the video feed at the point of sampling allows a further quality control check on the process especially in relation to outlier values (for example for atypical ground conditions at that point). The system can acquire soil sample locations while off-line.

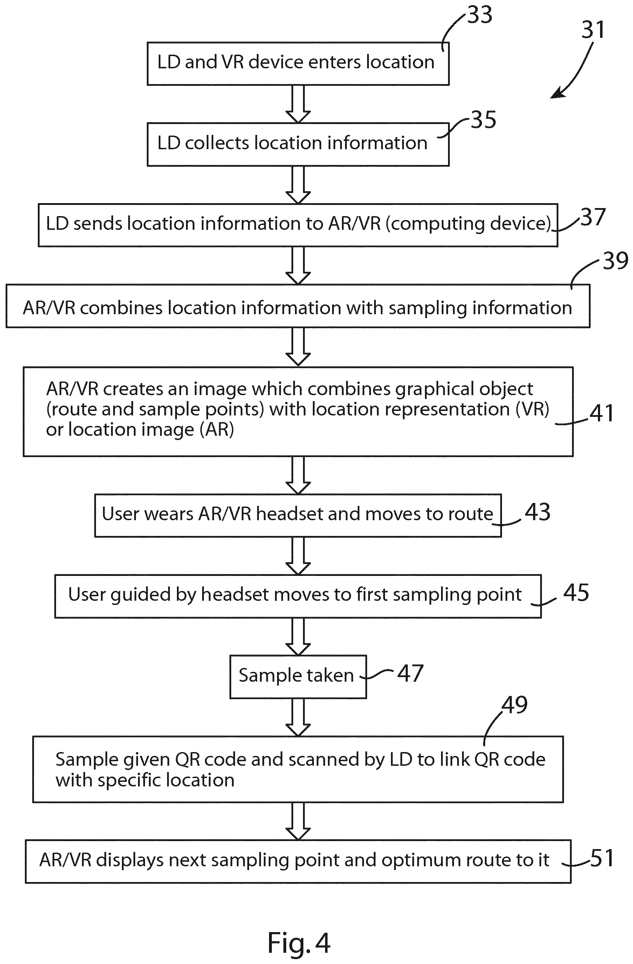

Figure 4 shows an example of the method 31 of the present invention as applied to the collection of soil samples. In this example of the present invention, the location device (LD, in this example a smartphone) and the VR device enters location 33 when brought into the location by a user.

Once in the location the LD collects location information 35 such as GPS information. The LD sends location information to the VR (computing device) 37 which combines the location information with sampling information 39 The VR device creates an image 41 which combines a graphical object (route and sample points) with the location representation which is shown on a VR device. In an alternative embodiment where an augmented reality device is used, the graphical object is combined with a location image or video.

Once the information on location and a representation of the location are displayed upon the GUI of the VR device, the user wears AR/VR headset and moves around the graphical representation of the pathway which defines the optimum or preferred route 43. The user then moves to first sampling point 45, a sample is taken 47. The sample is placed in a sample holder which has a QR code and is scanned by the location device to link QR code with specific location 49. The VR device then displays next sampling point and optimum route to it 51 until the full set of samples has been taken and logged.

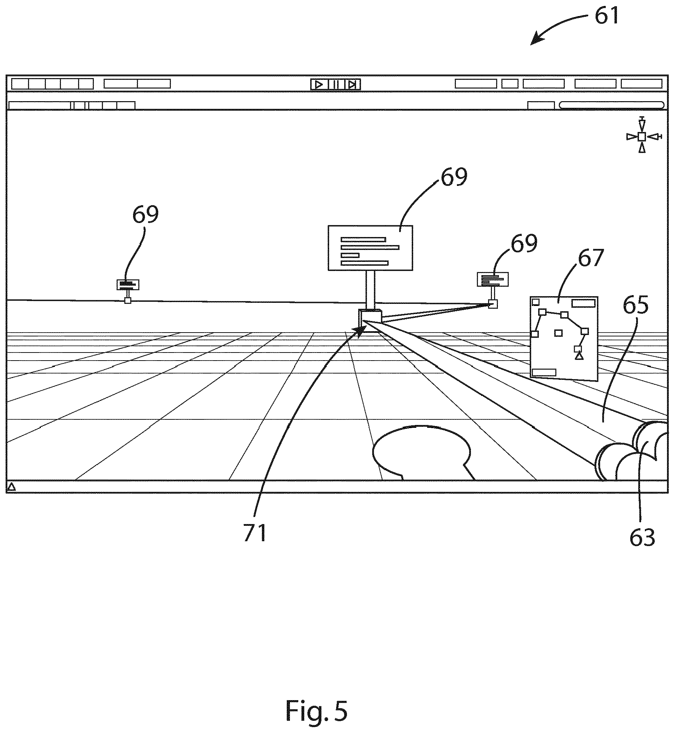

Figure 5 shows an example of a graphical user interface 61 as used in at least one embodiment of the present invention. The GUI shows the user/device location 63, the optimum/preferred/designated device path 65, a graphic inset 67 which shows the overall device path, soil parameter information 69 as a pop-up and the next sampling location 71.

Figure 5 shows the manner in which a route of POIs are plotted using in one embodiment red cubes. The invention further comprises a system for the in-situ visualisation of soil parameters at any POI as illustrated also in Fig. 5. The user may click on any POI in the route map and a corresponding graphical object will be displayed showing the soil parameters 69 at that point which are already in the back end of the system or obtained from sensors.

In addition to the use of this invention for soil sampling, another embodiment of it may be its use for other forms of navigation in-field by a farmer. Thus, the system may also be applied for creating and following a route back to a specific location whose location had previously been captured. For example, this might be an area of the field showing crop stress due to biotic or abiotic factors or disease outbreak. Utilising an API in a larger digital agriculture application, the user may then retrieve in visual form, information relevant to that particular location and/or record observations or work flow creation relevant to that location.

Advantageously, the soil sampling method in accordance with the present invention is used in precision agriculture in order to establish field management zones for variable rate applications.

The description of the invention including that which describes examples of the invention with reference to the drawings may comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. CD ROM, or magnetic recording medium, e.g. a memory stick or hard disk. The carrier may be an electrical or optical signal which may be transmitted via an electrical or an optical cable or by radio or other means.

The description of the invention including that which describes examples of the invention describes the use of virtual reality (VR) and augmented reality (AR) systems and apparatus. Herein, the term virtual reality is used to describe systems which immerses the user in an artificial digital environment.

VR headsets completely take over the user's vision to give the user the impression that they are somewhere else. Examples include the HTC Vive and the Oculus Rift. Such headsets are designed to completely block out the user's surroundings when worn.

AR overlays virtual objects onto the real-world environment. AR devices like the Microsoft HoloLens and various enterprise-level "smart glasses" are transparent, letting you see everything in front of you as if you are wearing a weak pair of sunglasses. The technology is designed for completely free movement while projecting images over whatever you look at. The term mixed reality overlays and anchors virtual objects to the real world.

In the specification the terms "comprise, comprises, comprised and comprising" or any variation thereof and the terms include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A method for determining physical attributes of a geographical area of interest, the method being **characterized by** the steps of:
determining the physical location of the area of interest;
creating a representation of the area of interest on a graphical user interface of a portable computing device;
mapping a graphical object onto a location in the representation of the area of interest, the graphical object representing a location in the area of interest where a task is to be undertaken; and
recording the performance of the task.

2. The method as claimed in claim 1 wherein, the geographical area is a field.

3. The method as claimed in claim 1 or claim 2 wherein, the location of the field is determined using Global Positioning System (GPS) location data which is obtained by the smartphone.

4. The method as claimed in any preceding claim wherein GPS data is made available to the headset via a wireless communication system such as Bluetooth®.

5. The method as claimed in claims 1 to 4 wherein, after an initial GPS "registration", location of the user is tracked using the graphical user interface of the computing device.

6. The method as claimed in any preceding claim wherein , the step of measuring the physical attributes comprises soil sampling.

7. The method as claimed in claim 3, wherein, the step of creating a representation of the area of interest on a graphical user interface of a portable computing device comprises acquiring the GPS position of the area of interest.

8. The method as claimed in any preceding claim wherein, the step of creating a representation of the area of interest on a graphical user interface comprises the creation of points of interest within the area of interest.

9. The method as claimed in any preceding claim wherein, the location of the points of interest is calculated to define optimum locations in the geographical area at which the task is undertaken.

10. The method as claimed in any preceding claim wherein the points of interest are sampling points where a soil sample is to be taken.

11. The method as claimed in any preceding claim wherein, the graphical object is mapped onto the points of interest.

12. The method as claimed in claim 11 wherein, the graphical object is mapped onto the points of interest to create a graphical representation of the points of interest which overlays the representation of the area of interest.

13. The method as claimed in any preceding claim wherein, the graphical object represents the position on the ground to which the device must be moved and from where the task must be performed.

14. The method as claimed in any preceding claim wherein, the performance of the task is recorded using a mobile tag which links the performed task with the location at which the task was performed.

15. A device for determining physical attributes of a geographical area of interest as defined in the method of the first aspect, the device being **characterized by**:
a location detector for determining the physical location of the area of interest;
a portable computing device which
creates a representation of the area of interest on a graphical user interface of a portable computing device based on the physical location determined by the location detector;
creates a mapping of a graphical object onto a location in the representation of the area of interest, the graphical object representing a location in the area of interest where a task is to be undertaken; and
a data recorder for recording the performance of the task.
